# EUROPEAN PATENT SPECIFICATION

(11) **EP 2 842 580 B1**
(45) Date of publication and mention of the grant of the patent: **25.09.2019**
(21) Application number: 13782055.1
(22) Date of filing: 18.04.2013
(51) Int. Cl.: A61L 15/28, D01D 1/02, A61F 13/00, A61L 15/46, B65B 5/04, B65B 55/12, D01F 1/10, D01F 9/00, D01F 9/04

(54) **SILVERY ANTIBACTERIAL FIBRE, TEXTURE, AND WOUND DRESSING, AND PREPARATION METHOD THEREOF**
SILBERNE ANTIBAKTERIELLE FASER, TEXTUR UND WUNDVERBAND SOWIE HERSTELLUNGSVERFAHREN DAFÜR
FIBRE ANTIBACTÉRIENNE ARGENTÉE, TEXTURE, PANSEMENT POUR PLAIE, ET PROCÉDÉ POUR LES PRÉPARER

(30) Priority: 23.04.2012 CN 201210122966
(43) Date of publication of application: 04.03.2015
(73) Proprietor: Foshan United Medical Technologies Ltd, Guangdong 528225 (CN)
(72) Inventor: WANG, Xiaodong, Foshan Guangdong 528225 (CN); TAO, Bingzhi, Foshan Guangdong 528225 (CN); MO, Xiaohui, Foshan Guangdong 528225 (CN); XU, Haitao, Foshan Guangdong 528225 (CN)
(74) Representative: Wilson Gunn
(86) International application number: PCT/CN2013/074345
(87) International publication number: WO 2013/159668

(56) References cited:
- EP-A1- 1 849 464
- EP-A1- 1 859 816
- EP-A2- 0 072 680
- WO-A1-01/24839
- WO-A1-2007/057657
- WO-A2-2009/141633
- CN-A- 1 833 731
- CN-A- 1 833 731
- CN-A- 101 450 222
- CN-A- 101 804 218

## Description

### Technical field

This invention relates to an antimicrobial fiber, fabric and wound dressing containing silver, and its method of manufacturing. The said wound dressing can release silver ions continuously to the wound site, providing an effective antibacterial function, and preventing wound infections from bacteria and other microorganisms. It is suitable for the management of chronic wounds.

### Background art

At present, the most common antimicrobial silver-containing wound dressings can be divided into 2 groups, i.e. metallic and ionic. The metallic silver dressing can be manufactured by coating the silver onto the fiber surface or by adding the silver metal such as nano silver into the fiber structure, while ionic silver dressings can be achieved by adding the silver compounds into the fiber structure. Both can release silver ions, killing the microorganisms when in contact with moisture or water.

CN1895683A describes a nano silver antibacterial dressing and its manufacturing method. The dressing is obtained by spraying the nano silver solution onto fiber surfaces. The dressing can contain 0.05-2.9% silver.

CN1066783A describes an antibacterial dressing combining nano silver. This dressing is obtained by vapor disposition of nano metals such as silver, copper and other alloys onto the fiber surface.

US7462753 describes a nano silver dressing which consists four layers. The first layer is a hydrophilic fabric; the second layer is an activated carbon fabric with nano silver; the third layer is a super absorbent nonwoven pad, and the fourth layer is a porous fabric.

US7385101 describes an antibacterial wound dressing suitable for the management of chronic wounds. The dressing is a nonwoven fabric composed of silver-coated fiber and alginate fiber.

EP1095179 describes a manufacturing method for nonwoven wound dressing, in which the silver-coated fiber is laminated with alginate nonwoven from both sides.

CN1673425A describes the manufacturing method for antimicrobial viscose fibers containing 0.1-1% nano silver. The nano silver is added to the spinning solution during the manufacture. The patent aims to introduce the silver metal particles into the fiber structure. The metallic silver releases very small amount of silver ions when in contact with water, compared to the ionic silver, therefore it requires a large amount of silver metal content in the fiber structure to meet antimicrobial requirements.

US6897349 and EP1216065 describe a method by which fibers are treated with a silver chloride solution making the material containing silver ions therefore becoming antibacterial.

US20030180346 and EP1318842 describe a silver wound dressing by blending silver fiber and non-silver fiber. This dressing contains 0.01-5% by weight silver.

CN1308509C describes a silver antibacterial chitosan dressing. In the dressing, silver sodium hydrogen zirconium phosphate (Alphasan) with 1um particle size is added into the spinning solution. The resultant fiber silver content is 3.0-4.0%.

EP1849464 and US2007275043 describe a method of manufacturing silver alginate fibers by adding silver carbonate into the spinning solution.

Ionic silver wound dressing has a very high utilization efficiency of silver, and is widely used in the advanced wound dressing industry. However, the ionic silver compound is also associated with low solubility; materials such as silver chloride and silver carbonate have difficulty dissolving in water, often resulting in a very small amount of silver ions being released before reaching equilibrium. Only when these sliver ions are depleted can more silver ions can be released again. This release-consumption-release cycle can provide the continuous release of silver ions, but, because of the low solubility, the amount of available silver ions is always small, therefore relatively large amounts of silver containing materials are needed in order to achieve a desired antimicrobial performance. WO2009/141633 discloses a method comprising dissolving silver nitrate in water and adding a polysaccharide such as alginate into the silver solution and further electrospinning said solution. A fabric is formed by needle punching and sterilized. Silver particles are present in the fibres from 0.002% to 2%. Silver is distributed evenly through the fibres so that a sustained release of silver is obtained from the dressing.

In order to address the above issues, this invention provides a method to add the silver ionic directly into the spinning solution by utilizing the bonding of the -COOH and -NH₂ to silver ions in the spinning solution of alginate and chitosan. By adding ionic silver compounds, preferably silver nitrate with 60% percent silver content, into the spinning solution uniformly, the utilization efficiency of silver ions can be increased to 70%.

In contrast with the method of dissolving water-insoluble silver compound particles, this patent application provides a method of adding water soluble silver nitrate solution into the spinning solution. With this method the silver ions can be distributed evenly across the entire volume of polymer solution and therefore to the fiber structure, providing a prolonged and effective antimicrobial performance. The duration of antimicrobial effect can be as long as 7 days.

In conclusion, this invention provides a method of uniformly distributing silver nitrate and silver ions evenly into the fiber structure, and a method of manufacturing the

### Disclosure of invention

The present invention mixes water soluble silver nitrate with spinning polymer solution, allowing silver ions to be distributed evenly across the entire fiber structure, enabling a more durable and faster release of silver ions on contact with water, and providing a long lasting antimicrobial efficiency i.e. 7 days.

The objective of this invention is to provide a silver antimicrobial fiber, fabric and wound dressing. The silver content of the silver fiber made from this invention, expressed as the percentage of the dry weight of the polymer, is 0.01-10%, preferably 0.1-7%, and most preferably 0.5-5%.

The polymer referred to in this invention is an alginate or a chitosan. The alginate can be a high Guluronic alginate, or a high Mannuronic alginate or a mixture of both. The alginate fiber can be calcium alginate fiber or sodium/calcium alginate fiber. The chitosan fiber shall have a degree of deacetylation of at least 80%. The chitosan fiber can also be chemically modified, such as by undergoing carboxymethylation or acylation process, in order to improve its gelling and absorbency. The alginate or chitosan fibers shall have a certain linear density and fiber length. The fiber linear density shall be 1 to 5 dtex, and the fiber length 5 to 125mm.

The said wound dressing is made through a needle punching nonwoven process or chemical bonding nonwovens process or weaving or knitting process. The fiber can be slightly longer, e.g. 30-100mm, if a needle punching nonwoven process is involved. The fiber can be slightly shorter, e.g. 3-15mm, if a chemical bonding nonwoven process is involved. Accordingly, the fiber length can be 20-85mm if a weaving or knitting process is employed. When the said silver wound dressing is made through the needle
The fiber can be slightly shorter, e.g. 3-15mm, if a chemical bonding nonwoven process is involved. Accordingly, the fiber length can be 20-85mm if a weaving or knitting process is employed. When the said silver wound dressing is made through the needle punching nonwoven process, its absorbency for a solution is 1200% or above, Dressing wet strength in machine direction (MD) is 0.3N/cm or above, Cross Machine Direction (CD) 0.4N/cm or above.

The second objective of this invention is to provide a method of manufacturing silver fiber and the silver wound dressing, which includes the following steps:
a) Dissolve the silver nitrate into the water;
b) Add the polymer, e.g. sodium alginate or chitosan into the above silver solution to obtain the silver containing polymer spinning solution. The ratio between the weight of silver ions and the dry weight of the polymer is between 0.01-10%, preferably 0.1-0.7%, most preferably 0.5-5%;
c) Extrude the above spinning solution into the silver antimicrobial fiber through the respective wet spinning process, this makes the silver fiber;
d) Convert the silver fiber into fabric through the needle punching nonwoven process, chemical bonding nonwovens process or weaving process or knitting process;
e) Cut, pack, sterilise the fabric to obtain the silver wound dressing.

Sodium hypochlorite is added into the silver nitrate solution between steps a) and b). The weight of sodium hypochlorite added shall be 0.005-2% of the polymer. Alternatively sodium chloride is added into the silver nitrate solution between steps a) and b). The weight sodium chloride added shall be 0.001%-11% of the polymer.

Therefore this invention provides a method of manufacturing the said antimicrobial wound dressing which includes the following steps:
a) Dissolve the silver nitrate into the water;
b) Add sodium hypochlorite into the silver nitrate solution. The weight of sodium hypochlorite added shall be 0.005-2% of the polymer;
c) Add the polymer, e.g. sodium alginate or chitosan into the above silver solution to obtain the silver containing polymer spinning solution. The ratio between the weight of silver ions and the dry weight of the polymer is between 0.01-10%;
d) Extrude the above spinning solution into the silver antimicrobial fiber through respective wet spinning process;
e) Convert the silver fiber into fabric through the needle punching nonwoven process, chemical bonding nonwovens process or weaving process or knitting process;
f) Cut, pack, sterilise the fabric to obtain the silver wound dressing.

Alternatively, this invention provides another method of manufacturing the said antimicrobial wound dressing which includes the following steps:
a) Dissolve the silver nitrate into the water;
b) Add sodium chloride into the silver nitrate solution. The weight of sodium chloride added shall be 0.001-11% of the polymer;
c) Add the polymer, e.g. sodium alginate or chitosan into the above silver solution to obtain the silver containing polymer spinning solution. The ratio between the weight of silver ions and the dry weight of the polymer is between 0.01-10%;
d) Extrude the above spinning solution into the silver antimicrobial fiber through the respective wet spinning process;
e) Convert the silver fiber into fabric through needle punching nonwoven process, chemical bonding nonwovens process or weaving process or knitting process;
f) Cut, pack, sterilise the fabric to obtain the silver wound dressing.

Moreover, in this invention the silver nitrate is mixed in water before adding the polymer material into the mix, this ensures that the silver nitrate is fully dissolved and mixed in water, then distributed uniformly into the entire polymer solution.

When the solution is extruded into fiber, and made into the wound dressing, the silver ions are also uniformly distributed in the structure of the fiber and the dressing.

When the wound dressing is in contact with water or wound fluid, the external surface of the fiber/dressing is moisturized first then releases silver ions first. When the water or wound fluid is further absorbed into the fiber/dressing structure, the silver ions in the inner structure of fiber/dressing can be further released, thus allowing a continuing and long lasting release of silver ions.

The manufacturing method for the antimicrobial fiber can be further improved. The improvement is made in the polymer mixing stage where a pre-mix of the polymer material in water is involved. At the start of the mixing, a small quantity of the polymer is mixed in water. The exact quantity of the pre-mix is such that can achieve a solution viscosity of 0.2-1 Pa.s (200-1000 cps). Then, whilst the solution is stirred continuously, silver nitrate is added into the mix. Preferably more polymer can be added to the solution so that an ideal viscosity of the mixed solution of 0.5-1 Pa.s (500-1000 cps) can be achieved. This viscosity can ensure a full mixing of silver nitrate without any grouping or aggregation silver material. Then the remaining polymer is added into the mix while the solution is being stirred continuously. Keep mixing for 20-90 mins then follow the steps of degassing and extrusion to manufacture the silver antimicrobial fibers. This invention provides a method of manufacturing silver fiber that is made by dissolving the silver nitrate directly into the polymer solutions of wet spinning process (such as alginate and chitosan) without using any reduction, stabilizing or dispersion agents in any steps of mixing or extrusion. The method is easy to use and with minimum cost.

As the silver ions are evenly distributed in said fibers and said wound dressing, the wound dressing of this design can provide continuous and long lasting release of silver ions thus is ideal for the management of chronic wounds, and can be used to prevent or reduce wound infections.

### Drawings

- Fig.1:: Zone of inhibition of the dressing containing 0.5% (weight) silver for *staphylococcus aureus* after 1 day;
- Fig.2:: Zone of inhibition of the dressing containing 0.5% (weight) silver for *staphylococcus aureus* after 5 days;
- Fig.3:: Zone of inhibition of the dressing containing 0.5% (weight) silver for staphylococcus aureus after 7 days;
- Fig.4:: Zone of inhibition of the dressing containing 1% (weight) silver for *escherichia coli* after 1 day;
- Fig.5:: Zone of inhibition of the dressing containing 1% (weight) silver for *escherichia coli* after 5 days;
- Fig.6:: Zone of inhibition of the dressing containing 1% (weight) silver for *escherichia coli* after 7 days;
- Fig.7:: Zone of inhibition of the dressing containing 10% (weight) silver for *bacillus subtilis* after 1 day;
- Fig.8:: Zone of inhibition of the dressing containing 10% (weight) silver for *bacillus subtilis* after 7days;
- Fig.9:: Zone of inhibition of the dressing containing 0.05% (weight) silver for *staphylococcus aureus* after 1 day;
- Fig.10:: Zone of inhibition of the dressing containing 0.05% (weight) silver for *staphylococcus aureus* after 7 days;
- Fig.11:: Zone of inhibition of the dressing containing 0.01% (weight) silver for *staphylococcus aureus* after 1 day;
- Fig.12:: Zone of inhibition of the dressing containing 0.01% (weight) silver for *staphylococcus aureus* after 7 days;

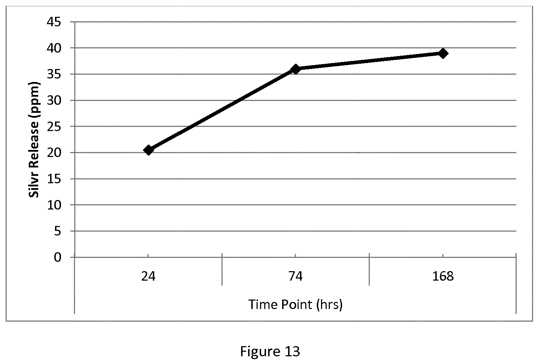

- Fig.13:: Silver releasing profile in 10ml simulated wound exudates of the dressing made from example 1.

### Example

This invention can be further illustrated through the following examples and figures.

The calculation for mixing and components weights can be summarized as follows:
For instance, the dry weight of sodium alginate powder is 6 kg, and the moisture content of the material is 11%, therefore the weight of sodium alginate at ambient conditions is: 6÷(1-11%) = 6.74kg. Normally, when preparing a polymer solution of sodium alginate at 5% solid content, the quantity of water needed for the mixing is 6÷5%×95%=114kg.

The silver content in silver nitrate is 60%. To make silver alginate fibers with 0.5% silver content, the weight of silver in 6 kg sodium alginate powder is calculated as: 6kg×0.5%=0.03kg, this requires 0.03÷60%=0.05kg of silver nitrate.

### Example 1 (not part of the invention)

The manufacturing method for antimicrobial fibers and wound dressing containing 0.5% by weight silver:
1. Add 114L of water into the mixing vessel;
2. To make 6 Kg of silver alginate fibers with 0.5% silver content, it will need 50g silver nitrate, 6.74kg sodium alginate and 114L water;
3. Add 50 g of silver nitrate into the mixing vessel which has been pre-charged with 114L of water. Start the mixer to fully dissolve and mix the silver nitrate in the water, then whilst the mixer is kept running add 1 kg sodium alginate powder into the solution, and keep the mixer running. Check the mixture for undissolved alginate and silver nitrate and ensure the viscosity reaches the pre-stated ideal level;
4. Add the remaining sodium alginate into the solution while the mixer is kept running;
5. After the sodium alginate is completely dispersed, put the solution on stand for 24 hours for natural degassing. Because of the high viscosity of the polymer solution, the silver ion is evenly distributed in the alginate polymer solution.
6. After the degassing is completed, the polymer solution is ready to be extruded into calcium silver alginate fiber through a standard wet-spinning process. Typically silver containing sodium alginate solution is pumped through a spinneret into a coagulating bath to convert the sodium alginate into calcium alginate fiber, and then followed with a stretching bath, washing, drying, crimping and cutting.
7. This will make white or off-white fibers with 0.5% (weight) silver content;
8. The silver fibers are converted into nonwoven felt, and cut into 10x10 cm pads for packaging. The dressing is irradiated by gamma at 25-40 kGy dosage.
9. The silver alginate dressing with 0.5% silver content is obtained.

### Example 2 (not part of the invention)

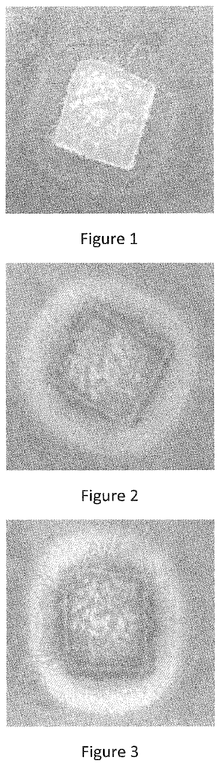

The dressing from example 1 is cut into 2x2 cm, and wetted and then placed into a petri dish that is covered evenly with staphylococcus aureus. The petri dish is placed into a 37°C incubator for 7 days, and observed for growth of microorganisms. When the silver ions are released from the dressing, the microorganisms surrounding the dressing sample are killed, creating a visible zone of inhibition. The greater the zone, the better the antimicrobial property the dressing has. Figure 1 displays a zone of inhibition of the dressing containing 0.5% (weight) silver for *staphylococcus aureus* after 1 day; Figure 2 displays a zone of inhibition of the dressing containing 0.5% (weight) silver for *staphylococcus aureus* after 5 days; Figure 3 displays a zone of inhibition of the dressing containing 0.5% (weight) silver for *staphylococcus aureus* after 7 days. It can be seen that the dressing with 0.5% silver content still has a good antimicrobial function after 7 days.

### Example 3 (not part of the invention)

The manufacturing method for antimicrobial fibers and wound dressing containing 1% by weight silver:
1. Add 114L of water into the mixing vessel;
2. To make 6 Kg of silver alginate fibers with 1% silver content, it will need 100g silver nitrate, 6.74kg sodium alginate and 114L water;
3. Add 100 g of silver nitrate into the mixing vessel which has been pre-charged with 114L of water. Start the mixer to fully dissolve and mix the silver nitrate in the water then whilst the mixer is kept running add 1 kg sodium alginate powder into the solution, and keep the mixer running. Check the mixture for undissolved alginate and silver nitrate and ensure the viscosity reaches the ideal level;
4. Add the remaining sodium alginate into the solution while the mixer is kept running;
5. After the sodium alginate is completely dispersed, keep the solution on stand still for 24 hours for natural degassing. Because of the high viscosity of the polymer solution, the silver ion is evenly distributed in the alginate polymer solution.
6. After the degassing is completed, the polymer solution is ready to be extruded into calcium silver alginate fiber through a standard wet-spinning process, typically silver containing sodium alginate solution is pumped through a spinneret into a coagulating bath to convert the sodium alginate into calcium alginate fiber, and then followed with stretching bath, washing, drying, crimping and cutting.
7. This will make white or off-white fibers with 1% (weight) silver content;
8. The silver fibers are converted into nonwoven felt, and cut into 10x10 cm pad for package. The dressing is irradiated by gamma at 25-40 kGy dosage.
9. The silver alginate dressing with 1% silver content is obtained.

### Example 4 (not part of the invention)

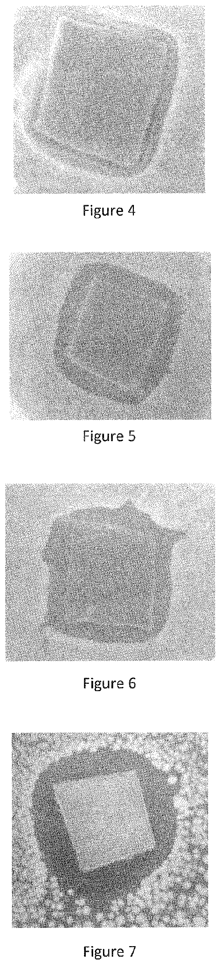

The dressing from example 3 is cut into 2x2 cm, and wetted and placed into a petri dish that is covered evenly with *escherichia coli.* The petri dish is placed into a 37°C incubator for 7 days, and observed for growth of microorganisms. When the silver ions are released from the dressing, the microorganisms surrounding the dressing sample are killed, creating a visible zone of inhibition. Figure 4 displays a zone of inhibition of the dressing containing 1% (weight) silver for *escherichia coli* after 1 day; Figure 5 displays a zone of inhibition of the dressing containing 1% (weight) silver for *escherichia coli* after 5 days; Figure 6 displays a zone of inhibition of the dressing containing 1% (weight) silver for *escherichia coli* after 7 days. It can be seen that the dressing with 1% silver content has a very good antimicrobial function after 7 days.

### (not part of the invention)

The manufacturing method for antimicrobial fibers and wound dressing containing 10% by weight silver:
1. Add 114L of water into the mixing vessel;
2. To make 6 Kg of silver alginate fibers with 10% silver content, it will need 1000g silver nitrate, 6.74kg sodium alginate and 114L water;
3. Add 1000 g of silver nitrate into the mixing vessel which has been pre-filled with 114L of water. Start the mixer to fully dissolve and mix the silver nitrate in the water, then whilst the mixer is kept running add 1 kg sodium alginate powder into the solution, and keep the mixer running. Check the mixture for undissolved alginate and silver nitrate and ensure the viscosity reaches the ideal level to prevent re-grouping of the silver material;
4. Add the remaining sodium alginate into the solution while the mixer is kept running;
5. After the sodium alginate is completely dispersed, keep the solution on stand still for 24 hours for natural degassing. Because of the high viscosity of the polymer solution, the silver ion is kept suspended and evenly distributed in the alginate polymer solution.
6. After the degassing is completed, the polymer solution is ready to be extruded into calcium silver alginate fiber through a standard wet-spinning process, typically silver containing sodium alginate solution is pumped through a spinneret into a coagulating bath to convert the sodium alginate into calcium alginate fiber, and then followed with stretching bath, washing, drying, crimping and cutting.
7. This will make white or off-white fibers with 10% (weight) silver content;
8. The silver fibers are converted into nonwoven felt, and cut into 10x10 cm pad for package. The dressing is irradiated by gamma at 25-40 kGy dosage.
9. The silver alginate dressing with 10% silver content is obtained.

### Example 6 (not part of the invention)

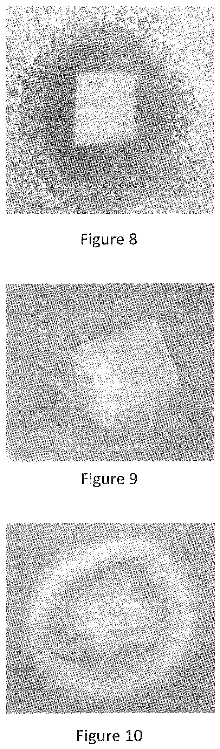

The dressing from example 5 is cut into 2x2 cm, and wetted and then placed into a petri dish that is covered evenly with *staphylococcus aureus.* The petri dish is placed into a 37°C incubator for 7 days, and observed for growth of microorganisms. When the silver ions are released from the dressing, the microorganisms surrounding the dressing sample are killed, creating a visible zone of inhibition. Figure 7 displays a zone of inhibition for *staphylococcus aureus* after 1 day; Figure 8 displays a zone of inhibition after 5 days; Figure 9 displays a zone of inhibition after 7 days. It can be seen that the dressing produces an excellent zone of inhibition 7 days.

### Example 7 (not part of the invention)

The manufacturing method for antimicrobial fibers and wound dressing containing 0.05% by weight silver:
1. Add 114L of water into the mixing vessel.
2. To make 6 Kg of silver alginate fibers with 0.05% silver content, it will need 5g silver nitrate, 6.74kg sodium alginate and 114L water.
3. Add all the silver nitrate into the mixing vessel which has been pre-filled with 114L of water. Start the mixer to fully dissolve and mix the silver nitrate in the water.
4. Add all the sodium alginate into the solution.
5. After the sodium alginate is completely dispersed, keep the solution on stand still for 24 hours for natural degassing. Because of the high viscosity of the polymer solution, the silver ion is evenly distributed in the alginate polymer solution.
6. After the degassing is completed, the polymer solution is ready to be extruded into calcium silver alginate fiber through a standard wet-spinning process.
7. This will make white or off-white fibers with 0.05% (by weight) silver content.
8. The silver fibers are converted into nonwoven felt, and cut into 10x10 cm pad for package. The dressing is irradiated by gamma at 25-40 kGy dosage.
9. The silver alginate dressing with 0.05% silver content is obtained.

### Example 8 (not part of the invention)

The dressing from example 7 is cut into 2x2 cm, and wetted and then placed into a petri dish that is covered evenly with *staphylococcus aureus.* The petri dish is placed into a 37°C incubator for 7 days, and observed for growth of microorganisms. When the silver ions are released from the dressing, the microorganisms surrounding the dressing sample are killed, creating a visible zone of inhibition. Figure 9 displays a zone of inhibition of dressing with 0.05% silver for *staphylococcus aureus* after 1 day; Figure 10 displays a zone of inhibition after 7 days. These suggest that the dressing with 0.05% of silver still has a reasonable good antimicrobial property.

### Example 9 (not part of the invention)

The manufacturing method for antimicrobial fibers and wound dressing containing 0.01% by weight silver:
1. Add 114L of water into the mixing vessel.
2. To make 6 Kg of silver alginate fibers with 0.01% silver content, it will need 1g silver nitrate, 6.74kg sodium alginate and 114L water.
3. Add all the silver nitrate into the mixing vessel which has been pre-filled with 114L of water. Start the mixer to fully dissolve and mix the silver nitrate in the water.
4. Add all the sodium alginate into the solution.
5. After the sodium alginate is completely dispersed, keep the solution on stand still for 24 hours for natural degassing. Because of the high viscosity of the polymer solution, the silver ion is evenly distributed in the alginate polymer solution.
6. After the degassing is completed, the polymer solution is ready to be extruded into calcium silver alginate fiber through a standard wet-spinning process.
7. This will make white or off-white fibers with 0.01% (by weight) silver content.
8. The silver fibers are converted into nonwoven felt, and cut into 10x10 cm pad for package. The dressing is irradiated by gamma at 25-40 kGy dosage.
9. The silver alginate dressing with 0.01% silver content is obtained.

### Example 10 (not part of the invention)

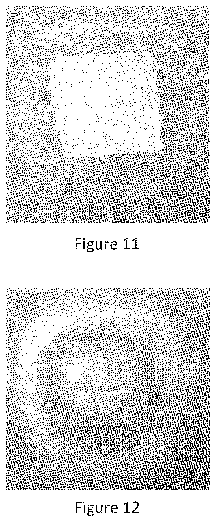

The dressing from example 9 is cut into 2x2 cm, and wetted and placed into a petri dish that is covered evenly with *staphylococcus aureus.* The petri dish is placed into a 37°C incubator for 7 days, and observed for growth of microorganisms. When the silver ions are released from dressing, the microorganisms surrounding the dressing sample are killed, creating a visible zone of inhibition. Figure 11 displays a zone of inhibition of dressing with 0.01% silver content for *staphylococcus aureus* after 1 day; Figure 12 displays a zone of inhibition after 7 days. These figures suggest that the dressing with 0.01% of silver still has some antimicrobial property.

### Example 11 (not part of the invention)

The manufacturing method for antimicrobial chitosan fibers and chitosan wound dressing containing 1.1% by weight silver:
1. Target silver content 1.1%, quantity of chitosan powder or flakes: 200 g, the moisture content of the chitosan is 10% by weight. At 5% (weight) solid content, 3420 ml of 2% (weight) acetic acid solution is needed. The dry weight of the chitosan powder is 180g.
2. To make 180g chitosan fiber with target silver content of 1.1%, 3.3g of silver nitrate is required.
3. Add all the silver nitrate into a small container that has pre-charged with the required amount of acetic acid solution, start the mixer to dissolve the silver nitrate.
4. Add 30 g of chitosan powder into the acetic acid solution prepared in the above step 3.
5. When the chitosan powder is fully dissolved and the solution reaches the ideal viscosity, add the remaining powder.
6. When all the chitosan is fully mixed into the solution, remove the mixer and leave the solution on stand still for 24 hours for natural degassing.
7. After the degassing is completed, the polymer solution is ready to be extruded into silver chitosan fiber through a standard wet-spinning process, typically silver containing chitosan solution is pumped through a spinneret into a bath of 5% (weight) sodium hydrate solution to convert the chitosan solution into filaments, and then followed with stretching bath, washing, drying, crimping and cutting.
8. This will make white or creamy colored fibers with 1.1% (by weight) silver content.
9. The silver fibers are converted into nonwoven felt on standard textile machine, and cut into 10x10 cm pad for package. The dressing is irradiated by gamma at 25-40 kGy dosage.
10. The silver chitosan dressing with 1.1% silver content is obtained.

### Example 12

The manufacturing method for antimicrobial fibers and wound dressing containing silver chloride:
1. Add 114L of water into the mixing vessel.
2. Add 5g of silver nitrate into the mixing vessel which has been pre-filled with 114L of water. Start the mixer to fully dissolve and mix the silver nitrate in the water. Add 1.72g sodium chloride to the solution whilst is mixer is kept running. The weight of sodium chloride is to maintain a molar ratio of 1:1 to silver nitrate. This will convert the silver nitrate into silver chloride. Add another 1 kg sodium alginate to the mix whilst the mixer is kept running.
3. Add the remaining 5.74kg sodium alginate to the solution whilst the mixer is on.
4. After the sodium alginate is completely dispersed, keep the solution on stand still for 24 hours for natural degassing. Because of the high viscosity of the polymer solution, the silver chloride is evenly mixed with the alginate polymer solution without any aggregation of silver chloride.
5. After the degassing is completed, the polymer solution is ready to be extruded into silver alginate fiber through a standard wet-spinning process, i.e. metering pump, coagulant bath, stretching, washing, drying, crimping and cutting.
6. This will make white or off-white silver alginate fibers with 0.05% (by weight) silver content.
7. The silver fibers are converted into nonwoven felt, and cut into 10x10 cm pad for package. The dressing is irradiated by gamma at 25-40 kGy dosage.
8. The silver alginate dressing with 0.05% silver content is obtained.

### Example 13

The manufacturing method for antimicrobial fibers and wound dressing containing silver hypochlorite:
1. Add 114L of water into the mixing vessel.
2. Add 5g of silver nitrate into the mixing vessel which has been pre-filled with 114L of water. Start the mixer to fully dissolve and mix the silver nitrate in the water. Add 2.19g sodium hypochlorite to the solution whilst is mixer is kept running. The weight of sodium hypochlorite is to maintain a molar ratio of 1:1 to silver nitrate. This will convert the silver nitrate into silver hypochlorite. Add another 1 kg sodium alginate to the mix whilst the mixer is kept running.
3. Add the remaining 5.74kg sodium alginate to the solution whilst the mixer is on.
4. After the sodium alginate is completely dispersed, keep the solution on stand still for 24 hours for natural degassing. Because of the high viscosity of the polymer solution, the silver hypochlorite is evenly mixed with the alginate polymer solution without any aggregation of silver chloride.
5. After the degassing is completed, the polymer solution is ready to be extruded into silver alginate fibers through a standard wet-spinning process, i.e. metering pump, coagulant bath, stretching, washing, drying, crimping and cutting.
6. This will make white or off-white silver alginate fibers with 0.05% (by weight) silver content.
7. The silver fibers are converted into nonwoven felt, and cut into 10x10 cm pad for package. The dressing is irradiated by gamma at 25-40 kGy dosage.
8. The silver alginate dressing with 0.05% silver content is obtained.

### Example 14

The manufacturing method for antimicrobial fibers and wound dressing containing silver chloride:
1. Add 114L of water into the mixing vessel.
2. Add 100g of silver nitrate into the mixing vessel which has been pre-filled with 114L of water. Start the mixer to fully dissolve and mix the silver nitrate in the water. Add 34.4g sodium chloride to the solution whilst is mixer is kept running. The amount of sodium chloride added is to maintain a molar ratio of 1:1 to silver nitrate. This will convert the silver nitrate into silver chloride. Add another 1 kg sodium alginate to the mix whilst the mixer is kept running.
3. Add the remaining 5.74kg sodium alginate to the solution whilst the mixer is on.
4. After the sodium alginate is completely dispersed, leave the solution stand still for 24 hours for natural degassing. Because of the high viscosity of the polymer solution, the silver chloride is evenly mixed with the alginate polymer solution without any aggregation.
5. After the degassing is completed, the polymer solution is ready to be extruded into silver alginate fiber through a standard wet-spinning process, i.e. metering pump, coagulant bath, stretching, washing, drying, crimping and cutting.
6. This will make white or off-white silver alginate fibers with 1.0% (by weight) silver content.
7. The silver fibers are converted into nonwoven felt, and cut into 10x10 cm pads for package. The dressing is irradiated by gamma at 25-40 kGy dosage.
8. The silver alginate dressing with 1.0% silver content is obtained.

### Example 15

The manufacturing method for antimicrobial fibers and wound dressing containing silver hypochlorite:
1. Add 114L of water into the mixing vessel.
2. Add 100g of silver nitrate into the mixing vessel which has been pre-filled with 114L of water. Start the mixer to fully dissolve and mix the silver nitrate in the water. Add 43.8g of sodium hypochlorite to the solution whilst is mixer is kept running. The amount of sodium hypochlorite is to maintain a molar ratio of 1:1 to silver nitrate. This will convert the silver nitrate into silver hypochlorite. Add another 1 kg sodium alginate to the mix whilst the mixer is kept running.
3. Add the remaining 5.74kg sodium alginate to the solution whilst the mixer is on.
4. After the sodium alginate is completely dispersed, leave the solution stand still for 24 hours for natural degassing. Because of the high viscosity of the polymer solution, the silver hypochlorite is evenly mixed with the alginate polymer solution without the formation of any aggregation.
5. After the degassing is completed, the polymer solution is ready to be extruded into silver alginate fibers through a standard wet-spinning process, i.e. metering pump, coagulant bath, stretching, washing, drying, crimping and cutting.
6. This will make white or off-white silver alginate fibers with 1% (by weight) silver content.
7. The silver fibers are converted into nonwoven felt, and cut into 10x10 cm pads for package. The dressing is irradiated by gamma at 25-40 kGy dosage.
8. The silver alginate dressing with 1% silver content is obtained.

### Example 16

The manufacturing method for antimicrobial fibers and wound dressing containing silver chloride:
1. Add 114L of water into the mixing vessel.
2. Add 1000g of silver nitrate into the mixing vessel which has been pre-filled with 114L of water. Start the mixer to fully dissolve and mix the silver nitrate in the water. Add 344g sodium chloride to the solution whilst is mixer is kept running. The amount of sodium chloride added is to maintain a molar ratio of 1:1 to silver nitrate. This will convert the silver nitrate into silver chloride. Add another 1 kg sodium alginate to the mix whilst the mixer is kept running. Check the viscosity.
3. Add the remaining 5.74kg sodium alginate to the solution whilst the mixer is on.
4. After the sodium alginate is completely dispersed, leave the solution on stand still for 24 hours for natural degassing. Because of the high viscosity of the polymer solution, the silver chloride is evenly mixed with the alginate polymer solution without any aggregation.
5. After the degassing is completed, the polymer solution is ready to be extruded into silver alginate fiber through a standard wet-spinning process, i.e. metering pump, coagulant bath, stretching, washing, drying, crimping and cutting.
6. This will make white or off-white silver alginate fibers with 10% (by weight) silver content.
7. The silver fibers are converted into nonwoven felt, and cut into 10x10 cm pads for package. The dressing is irradiated by gamma at 25-40 kGy dosage.
8. The silver alginate dressing with 10% silver content is obtained.

### Example 17

The manufacturing method for antimicrobial fibers and wound dressing containing silver hypochlorite:
1. Add 114L of water into the mixing vessel.
2. Add 1000g of silver nitrate into the mixing vessel which has been pre-filled with 114L of water. Start the mixer to fully dissolve and mix the silver nitrate in the water. Add 438g of sodium hypochlorite to the solution whilst is mixer is kept running. The amount of sodium hypochlorite is to maintain a molar ratio of 1:1 to silver nitrate. This will convert the silver nitrate into silver hypochlorite. Add another 1 kg sodium alginate to the mix whilst the mixer is kept running.
3. Add the remaining 5.74kg sodium alginate to the solution whilst the mixer is on.
4. After the sodium alginate is completely dispersed, leave the solution on stand still for 24 hours for natural degassing. Because of the high viscosity of the polymer solution, the silver hypochlorite is evenly mixed with the alginate polymer solution without any aggregation.
5. After the degassing is completed, the polymer solution is ready to be extruded into silver alginate fibers through a standard wet-spinning process, i.e. metering pump, coagulant bath, stretching, washing, drying, crimping and cutting.
6. This will make white or off-white silver alginate fibers with 10% (by weight) silver content.
7. The silver fibers are converted into nonwoven felt, and cut into 10x10 cm pads for package. The dressing is irradiated by gamma at 25-40 kGy dosage.
8. The silver alginate dressing with 10% silver content is obtained.

### Example 18

### Silver release

In order to establish the silver release profile of the silver containing antimicrobial wound dressing, the silver dressing from example 1 is cut into 2.5x2.5cm and placed into 10ml of simulated wound fluid. The sample is incubated in a water bath at 37°C, and kept shaking at 60-80rpm for 7 days. The silver ions will be released into the wound fluid and the amount of the silver in the solution is tested at the time points of 24hrs, 72hrs and 168 hrs. The following table (table 1) gives the amount of silver released into 10ml simulated wound exudates at the relevant time points. It can be seen that the silver release increases with the time, with the maximum silver release of 38.4ppm at the time point of 168hrs.

**Table 1 silver release at 10ml simulated wound exudate**

| Time point (hrs) | Silver release (ppm) |
|---|---|
| 24 | 20.3 |
| 72 | 36.1 |
| 168 | 38.4 |

## Claims

1. A method of manufacturing an antimicrobial wet-spun fibrous silver wound dressing comprising:
a) Dissolving silver nitrate in water;
b) Adding sodium chloride or sodium hypochlorite to the silver nitrate solution produced in step a);
c) Adding a polymer of required weight into the silver solution produced in step b) to make a silver containing polymer solution, the weight of silver ions to the dry weight of the polymer is 0.01-10% by weight;
d) Extruding the silver containing polymer solution into silver antimicrobial fibers through a wet spinning process;
e) Converting the silver fibers into fabrics through needle punching non-woven process, chemical bonding non-woven process, a weaving process or a knitting process;
f) Cutting, packing and sterilizing the dressing.

2. A method according to claim 1, **characterized in that** the said silver fiber is silver chitosan fiber or silver alginate fiber.

3. A method according to claim 2, **characterized in that** the said alginate fibers are high Guluronic alginate fibers, or high Mannuronic alginate fibers, or alginate fibers with equal blend of Guluronic and Mannuronic.

4. A method according to claim 3, **characterized in that** the said alginate fibers are calcium alginate fibers or calcium/sodium alginate fibers.

5. A method according to claim 2, **characterized in that** the said chitosan fiber has a degree of deacetylation of at least 80%.

6. A method according to claim 2, **characterized in that** said chitosan fibers are chemically modified by the carboxymethylation process or acylation process.

7. A method according to claim 1, **characterized in that** the said silver fiber has a linear density of 1-5dtex, fiber length of 5-125mm.

8. A method according to claim 1, **characterized in that** the said antimicrobial silver dressing is a needle punched nonwoven fabric with the absorbency to solution A being at least 1200%, the wet strength in machine direction being at least 0.3N/cm, the wet strength in cross machine direction being at least 0.4N/cm.

9. A method of manufacturing the antimicrobial wet-spun fibrous silver wound dressing according to claim 1, **characterized in that** sodium hypochlorite is added in step b), the weight ratio of sodium hypochlorite to that of polymer being between 0.005% to 2%.

10. A method of manufacturing the antimicrobial wet-spun fibrous silver wound dressing according to claim 1, **characterized in that** sodium chloride is added in step b), the weight ratio of sodium chloride to that of polymer being between 0.001% to 11.0%.

11. A method of manufacturing the antimicrobial wet-spun fibrous silver wound dressing according to claim 1, **characterized in that** a small portion of the required polymer is pre-mixed in water so that the viscosity of the pre-mixed solution is between 200-1000 cps, then the silver nitrate is added to the solution whilst the mixer is kept running, continue the mixing for another 20-90 minutes before adding the remaining polymer to the solution, followed by degassing and extrusion into fibers.

## Patentansprüche

1. Verfahren zum Herstellen eines antimikrobiellen nassgesponnenen fasrigen Silberwundverbandes aufweisend:
a) Auflösen von Silbernitrat in Wasser;
b) Hinzufügen von Natriumchlorid oder Natriumhypochlorid zu der Silbernitratlösung, welche in Schritt 1 hergestellt wurde;
c) Hinzufügen eines Polymers mit erforderlichem Gewicht in die Silberlösung, welche in Schritt b) hergestellt wurde, um eine silberaufweisende Polymerlösung herzustellen, wobei das Gewicht der Silberionen zu dem Trockengewicht des Polymers 0,01 bis 10 Gew.-% ist;
d) Extrudieren der silberaufweisenden Polymerlösung in antimikrobielle Silberfasern mittels eines Nassspinnprozesses;
e) Umwandeln der Silberfasern in Gewebe durch einen nicht-webenden Vernadelungsprozess, einen nicht-webenden chemischen Bindungsprozess, einen webenden Prozess oder einen strickenden Prozess;
f) Schneiden, Verpacken und Sterilisieren des Verbandes.

2. Verfahren nach Anspruch 1,
**dadurch gekennzeichnet,**
**dass** die Silberfasern Silberchitosanfasern oder Silberalginatfasern sind.

3. Verfahren nach Anspruch 2,
**dadurch gekennzeichnet,**
**dass** die Alginatfasern hochguluronische Alginatfasern oder hoch mannurovische Alginatfasern oder Alginatfasern mit einer gleichen Mischung von Guluron und Mannuron sind.

4. Verfahren nach Anspruch 3,
**dadurch gekennzeichnet,**
**dass** die Alginatfaser Kalziumalginatfasern oder Kalzium-/Natriumaginatfasern sind.

5. Verfahren nach Anspruch 2,
**dadurch gekennzeichnet,**
**dass** die Chistosanfaser einen Deacetylierungsgrad von mindestens 80% aufweisen.

6. Verfahren nach Anspruch 2,
**dadurch gekennzeichnet,**
**dass** die Chitosanfasern chemisch modifiziert durch das Carboxymetilierungsverfahren oder das Axylierungsverfahren sind.

7. Verfahren nach Anspruch 1,
**dadurch gekennzeichnet,**
**dass** die Silberfaser eine lineare Dichte von 1 bis 5 dtex und eine Faserlänge von 1 bis 125 mm aufweist.

8. Verfahren nach Anspruch 1,
**dadurch gekennzeichnet,**
**dass** der antimikrobielle Verband ein vernadeltes nicht-gewebtes Gewebe mit einem Absorptionsvermögen für eine Lösung A um mindestens 1200%, wobei die Nassfestigkeit in Maschinenrichtung mindestens 0,3 N/cm, und die Nassfestigkeit in Maschinenquerrichtung mindestens 0,4 N/cm beträgt.

9. Verfahren zum Herstellen des antimikrobiellen nassgesponnenen fasrigen Silberwundverbandes nach Anspruch 1,
**dadurch gekennzeichnet,**
**dass** Natriumhypochlorid im Schritt b) hinzugefügt wird, wobei das Gewichtsverhältnis von Natriumhypochlorid zu dem des Polymers zwischen 0,005% bis 2% liegt.

10. Verfahren zum Herstellen des antimikrobiellen nassgesponnenen fasrigen Silberwundverbandes nach Anspruch 1,
**dadurch gekennzeichnet,**
**dass** Natriumchlorid in Schritt b) hinzugefügt wird, wobei das Gewichtsverhältnis von Natriumchlorid zu dem des Polymers zwischen 0,001% und 11,0% liegt.

11. Verfahren zum Herstellen des antimikrobiellen nassgesponnenen fasrigen Silberwundverbandes nach Anspruch 1,
**dadurch gekennzeichnet,**
**dass** ein kleiner Teil des benötigten Polymers in Wasser vorgemischt wird, so dass die Viskosität der vorgemischten Lösung zwischen 200 bis 1000 cps liegt, dann das Silbernitrat hinzugefügt wird während der Mischer läuft, wobei das Mischen für weitere 20 bis 90 Minuten fortgeführt wird, bevor das restliche Polymer der Lösung zugefügt wird, folgend von Entgasen und Extrudieren zu Fasern.

## Revendications

1. Procédé de fabrication d'un pansement pour plaie en argent fibreux antimicrobien filé par voie humide comprenant :
a) la dissolution de nitrate d'argent dans de l'eau ;
b) l'ajout de chlorure de sodium ou d'hypochlorite de sodium à la solution de nitrate d'argent produite à l'étape a) ;
c) l'ajout d'un polymère de poids requis à la solution d'argent produite à l'étape b) pour créer une solution polymère contenant de l'argent, le poids d'ions d'argent sur le poids sec du polymère est de 0,01-10% en poids ;
d) l'extrusion de la solution polymère contenant de l'argent en fibres antimicrobiennes en argent par le biais d'un processus de filage par voie humide ;
e) la conversion des fibres d'argent en tissus par le biais d'un traitement de non-tissé par aiguilletage, d'un traitement de non-tissé par liaison chimique, d'un traitement de tissage ou d'un traitement de tricotage ;
f) la découpe, l'emballage et la stérilisation du pansement.

2. Procédé selon la revendication 1, **caractérisé en ce que** ladite fibre d'argent est une fibre de chitosane d'argent ou une fibre d'alginate d'argent.

3. Procédé selon la revendication 2, **caractérisé en ce que** lesdites fibres d'alginate sont des fibres d'alginate à Guluronique élevé, ou des fibres d'alginate à Mannuronique élevé, ou des fibres d'alginate à mélange égal de Guluronique et de Mannuronique.

4. Procédé selon la revendication 3, **caractérisé en ce que** lesdites fibres d'alginate sont des fibres d'alginate de calcium ou des fibres d'alginate de calcium/sodium.

5. Procédé selon la revendication 2, **caractérisé en ce que** ladite fibre de chitosane a un degré de désacétylation d'au moins 80 %.

6. Procédé selon la revendication 2, **caractérisé en ce que** lesdites fibres de chitosane sont chimiquement modifiées par le processus de carboxyméthylation ou le processus d'acétylation.

7. Procédé selon la revendication 1, **caractérisé en ce que** ladite fibre d'argent a une densité linéaire de 1-5 dtex, une longueur de fibre de 5-125 mm.

8. Procédé selon la revendication 1, **caractérisé en ce que** ledit pansement d'argent antimicrobien est un tissu non tissé aiguilleté avec une capacité d'absorption à la solution A d'au moins 1200 %, une résistance à l'état humide dans le sens machine d'au moins 0,3 N/cm, et une résistance à l'état humide dans le sens transversal à la machine d'au moins 0,4 N/cm.

9. Procédé de fabrication du pansement pour plaie en argent fibreux antimicrobien filé par voie humide selon la revendication 1, **caractérisé en ce que** l'hypochlorite de sodium est ajouté à l'étape b), le rapport en poids d'hypochlorite de sodium sur celui du polymère étant compris entre 0,005 % et 2%.

10. Procédé de fabrication du pansement pour plaie d'argent fibreux antimicrobien filé par voie humide selon la revendication 1, **caractérisé en ce que** le chlorure de sodium est ajouté à l'étape b), le rapport en poids de chlorure de sodium sur celui du polymère étant compris entre 0,001 % et 11,0 %.

11. Procédé de fabrication du pansement pour plaie d'argent fibreux antimicrobien filé par voie humide selon la revendication 1, **caractérisé en ce qu'**une petite partie du polymère requis est pré-mélangée dans de l'eau de telle sorte que la viscosité de la solution pré-mélangée est comprise entre 200-1000 cps, puis le nitrate d'argent est ajouté à la solution alors que le mélangeur continue de tourner, le mélange se poursuit pendant encore 20-90 minutes avant que le reste du polymère ne soit ajouté à la solution, suivi du dégazage et de l'extrusion en fibres.
